(19) Europäisches Patentamt
European Patent Office
Office européen des brevets

(11) **EP 4 678 092 A1**

(12) **EUROPEAN PATENT APPLICATION**

(43) Date of publication:
**14.01.2026 Bulletin 2026/03**

(21) Application number: **25188715.4**

(22) Date of filing: **10.07.2025**

(51) International Patent Classification (IPC):
**A61B 5/00** *(2006.01)*    **A61B 5/0538** *(2021.01)*
**A61B 5/06** *(2006.01)*    **A61B 18/14** *(2006.01)*

(52) Cooperative Patent Classification (CPC):
**A61B 5/0036; A61B 5/0538; A61B 5/066;**
**A61B 5/068; A61B 5/6852; A61B 5/6886;**
**A61B 18/1492;** A61B 5/287; A61B 5/367

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH LA MA MD TN**

(30) Priority: **11.07.2024  IL 31426824**
**02.06.2025  US 202519225052**

(71) Applicant: **Biosense Webster (Israel) Ltd.**
**2066717 Yokneam (IL)**

(72) Inventors:
• **COHEN, Benjamin**
**2066717 Yokneam (IL)**
• **KATZ, Natan Sharon**
**2066717 Yokneam (IL)**
• **BERMAN, Dror**
**2066717 Yokneam (IL)**
• **ROSENWEIG, Roni**
**2066717 Yokneam (IL)**

(74) Representative: **Carpmaels & Ransford LLP**
**One Southampton Row**
**London WC1B 5HA (GB)**

(54) **METHODS, SYSTEMS, AND GUI FOR ENHANCED REAL-TIME VISUAL FEEDBACK OF INTRALUMINAL CATHETER ENGAGEMENT**

(57)    The presently disclosed subject matter includes a computer system, method, and graphical user interface that provide graphical feedback indicating real-time contact level between a tissue wall of a luminal organ and an ablation catheter, based on electrical impedance sensed by electrodes of a catheter. As the impedance is related to proximity of the electrodes to the tissue walls, the system utilizes impedance measurements to visually alter the appearance of a graphical representation of the electrodes according to changes in the sensed impedance. The graphical feedback enables the physician to make real-time adjustments to the catheter's positioning and applied force, and thereby enhance the precision and effectiveness of intraluminal catheter therapy. The graphical feedback comprises a plurality of contact lobes centered on the electrodes and have sizes which increase with the contact level.

EP 4 678 092 A1

## Description

## TECHNOLOGICAL FIELD

The presently disclosed subject matter relates to visualization techniques for intraluminal catheter therapy in the diagnosis and treatment of medical disorders.

## BACKGROUND

Intraluminal catheter therapy (ICT) or catheterization has become a pivotal instrument in the field of medicine for both the diagnosis and treatment of various medical disorders. This minimally invasive procedure involves guiding a slender, flexible tube, or catheter, into a luminal organ. Equipped with electrodes, the catheter is used, *inter alia,* for mapping the organ, and identifying precise locations related to abnormal medical conditions.

Cardiac ICT, a particular example of ICT, is an important tool for both the diagnosis and treatment of cardiac disorders, particularly arrhythmias. This involves inserting a catheter equipped with electrodes through the blood vessels and into the heart, using the catheter for generating a map of the heart's electrical activity, and identifying the precise locations of abnormal electrical activity. The identified sites can then be treated through ablation, where targeted energy neutralizes the abnormal tissue, restoring normal heart rhythm. This integrated approach has revolutionized cardiac care, offering patients less invasive options with shorter recovery times.

## OVERVIEW

An important aspect of catheterization in the context of cardiology is related to tissue contact and ablation accuracy. Ensuring adequate contact between the catheter distal end assembly (e.g., balloon and/or basket) located at its distal end and the heart tissue is crucial for accurate and effective results.

A physician manipulating a catheter would benefit from knowing what a contact level of the catheter with a tissue wall is. In other words, how much force is being applied on the tissue wall. The force distribution on a distal end assembly can help the physician to accurately steer the end. Further, it is desired to avoid the application of excessive force on the tissue when manipulating the catheter, as this may result in deformation of the tissue surface, e.g., tenting, and consequently cause inefficient operation. An indication of force being applied on the tissue walls is also helpful during various other procedures such as Pulsed Field Ablation (PFA). Too little contact can render the ablation ineffective, while too much pressure can cause excessive tissue damage. An indication of the proximity between the catheter and tissue can help the physician reach a threshold level of force to achieve a desired lesion depth.

The presently disclosed subject matter includes a computer system, method, and graphical user interface that provide graphical feedback indicating real-time proximity based on electrical impedance sensed by electrodes of a catheter. As the impedance is related to proximity of the electrodes to the tissue walls, the system utilizes impedance measurements to visually alter appearance of a graphical representation of the electrodes according to changes in the sensed impedance. The graphical feedback enables the physician to make real-time adjustments to the catheter's positioning and applied force, and thereby enhance the precision and effectiveness of ICT. For instance, if the impedance suggests inadequate contact, the physician may reposition the catheter to achieve better engagement with the tissue. Conversely, a high impedance reading could signal excessive pressure, prompting the physician to reduce the contact force to prevent tenting and/or potential tissue damage.

A problem addressed by the current disclosure is achieving a balance between high-detail visualization and a sufficiently simple structure of visual elements. This issue is resolved in this disclosure by using 'contact lobes', formed by curves overlaid (superimposed) on the rendering of the catheter, and positioned at an optimal distance range. The size of these contact lobes indicates the level of contact. Additionally, the contact lobes are designed to be easily distinguishable from one another.

## BRIEF DESCRIPTION OF THE DRAWINGS

In order to better understand the subject matter that is disclosed herein and to exemplify how it may be carried out in practice, examples will now be described, by way of non-limiting example only, with reference to the accompanying drawings, in which:

FIG. 1A illustrates a catheter-based electrophysiology mapping and ablation system, according to examples of the presently disclosed subject matter;

FIG. 1B shows a flowchart illustrating a computer-implemented method according to embodiments of the present disclosure.

FIG. 1C schematically illustrates transverse and tangential directions, according to embodiments of the present disclosure.

FIG. 2A-2F schematically illustrate various contact lobes according to embodiments of the present disclosure.

FIG. 3A-3B schematically illustrate merging of non-adjacent contact lobes, according to embodiments of the present disclosure.

FIG. 3C-3E schematically illustrate an inner side according to embodiments of the present disclosure.

FIG. 4A schematically illustrates transverse and tangential radii, according to embodiments of the present disclosure.

FIG. 4B schematically illustrates bending of contact lobes, according to embodiments of the present disclosure.

**FIG. 5A-5D** show exemplary screenshots of visual output of methods and systems according to embodiments of the present disclosure.

**FIG. 6** shows a block diagram schematically illustrating a system according to embodiments of the present disclosure.

## DETAILED DESCRIPTION

In the following detailed description, numerous specific details are set forth in order to provide a thorough understanding. However, it will be understood by those skilled in the art that the presently disclosed subject matter may be practiced without these specific details. In other instances, well-known methods and features have not been described in detail so as not to obscure the presently disclosed subject matter.

Unless specifically stated otherwise, as apparent from the following discussions, it is appreciated that throughout the specification discussions utilizing terms such as "processing", "computing", "updating", "rendering" or the like, refer to the action(s) and/or process(es) of a computer that manipulate and/or transform data into other data, said data represented as physical, such as electronic, quantities and/or said data representing the physical objects. The term "computer" should be expansively construed to cover any kind of hardware-based electronic device with data processing capabilities. Processing capabilities may include processing circuitry. Each processing circuitry can comprise, for example, one or more processors operatively connected to (including non-transitory) computer memory, loaded with executable instructions for executing operations, as further described below .

The one or more processors referred to herein can represent for example, one or more general-purpose processing devices such as a microprocessor, a central processing unit, or the like. More particularly, a given processor may be one of: a complex instruction set computing (CISC) microprocessor, a reduced instruction set computing (RISC) microprocessor, a very long instruction word (VLIW) microprocessor, processor implementing other instruction sets, or a processor implementing a combination of instruction sets. The one or more processors may also be one or more special-purpose processing devices such as an application specific integrated circuit (ASIC), a field programmable gate array (FPGA), a digital signal processor (DSP), a graphics processing unit (GPU), a network processor, or the like.

The various illustrative logical blocks, modules, and algorithm steps described in connection with the examples disclosed herein can be implemented as electronic hardware, computer software, or combinations of both. To clearly illustrate this interchangeability of hardware and software, various illustrative components, blocks, modules, and steps are described generally in terms of their functionality. Whether such functionality is implemented as hardware or software depends upon the particular application and design constraints imposed on the overall system. The described functionality can be implemented in varying ways for each particular application, but such implementation decisions should not be interpreted as causing any departure from the scope of the disclosure.

It will also be understood that a system according to the present disclosure may be, at least partly, implemented on a suitably programmed computer. Likewise, the present disclosure contemplates a computer program being readable by a computer for executing the method of the present disclosure. The present disclosure further contemplates a non-transitory computer-readable memory tangibly embodying a program of instructions executable by the computer for executing the method of the present disclosure.

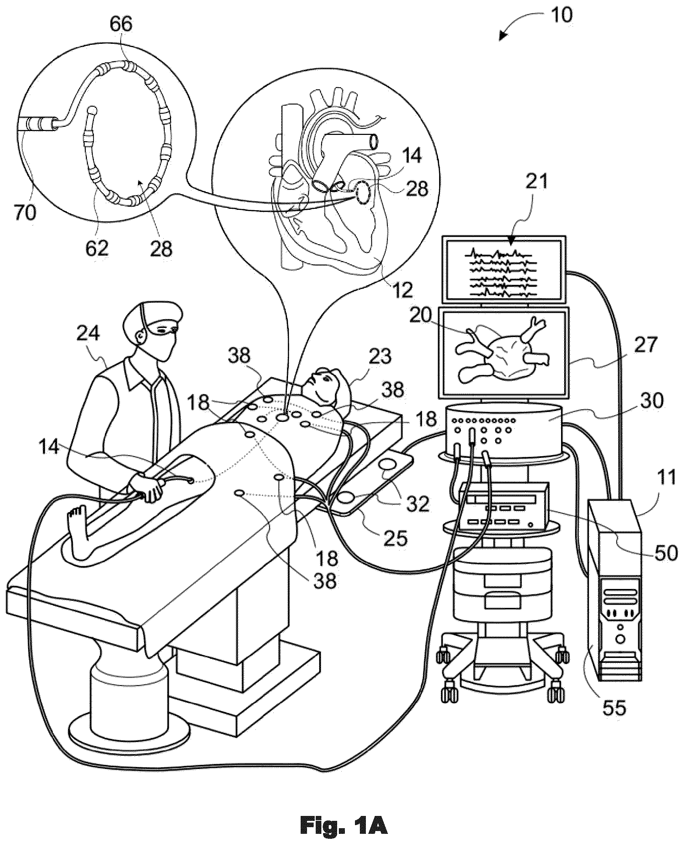

Reference is made to **FIG. 1A** showing an example catheter-based electrophysiology mapping and ablation system **10**. System **10** includes multiple catheters, which are percutaneously inserted by a physician **24** through the patient's vascular system into a chamber or vascular structure of a heart **12**. Typically, a delivery sheath catheter is inserted into the left or right atrium near a desired location in heart **12**. Thereafter, the catheter may be inserted into the delivery sheath catheter so as to arrive at the desired location in the heart **12**.

The plurality of catheters may include catheters dedicated for sensing Intracardiac Electrogram (IEGM) signals, catheters dedicated for ablating and/or catheters dedicated for both sensing and ablating. An example catheter **14** that is configured for ablating tissue and/or for sensing electrical cardiac activity and/or mapping is illustrated herein. Physician **24** may place a distal tip **28** of catheter **14** in contact with the epicardial tissue surface for sensing and/or ablating.

Catheter **14** is an exemplary catheter that includes a distal assembly, having one and preferably multiple electrodes **66** optionally distributed over a plurality of frame elements **62** at distal tip **28**. The electrodes are generally configured for delivering ablation energy to tissue, and/or for sensing cardiac electrical signals. Catheter **14** additionally includes one or more position sensors **70** embedded in or near distal tip **28** for tracking position and orientation of distal tip **28**. Optionally and preferably, position sensor **70** is a magnetic based position sensor, for example a position sensor including three magnetic coils for sensing three-dimensional (3D) position and orientation; or a position sensor including one magnetic coil, for sensing a single direction.

Each of the magnetic based position sensors **70** may be operated together with a location pad **25** including a plurality of magnetic coils **32** configured to generate magnetic fields in a predefined working volume. Real time position of distal tip **28** of catheter **14** may be tracked based on magnetic fields generated with location pad **25** and sensed by magnetic based position sensor **70**. Details of the magnetic based position sensing technology are described in U.S. Patent Nos. 5,5391,199;

5,443,489; 5,558,091; 6,172,499; 6,239,724; 6,332,089; 6,484,118; 6,618,612; 6,690,963; 6,788,967; 6,892,091.

System **10** includes one or more electrode patches **38** positioned for skin contact on patient **23** to establish location reference for location pad **25** as well as impedance-based tracking of electrodes **66**. For impedance-based tracking, electrical current is directed to electrodes **66** and sensed at electrode skin patches **38** so that the location of each electrode can be triangulated via the electrode patches **38**. Details of the impedance-based location tracking technology are described in US Patent Nos. 7,536,218; 7,756,576; 7,848,787; 7,869,865; and 8,456,182.

A recorder **11** records and displays electrograms **21** captured with body surface ECG electrodes **18** and optionally cardiac signals captured with electrodes **66** of catheter **14**. Recorder **11** may include pacing capability for pacing the heart rhythm and/or may be electrically connected to a standalone pacer.

System **10** may include an ablation energy generator **50** that is adapted to conduct ablative energy to electrodes **66** of the distal assembly at a distal tip of the catheter. Energy produced by ablation energy generator **50** may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) **30** is an interface configured to establish electrical communication between the at least one catheter with other catheters, other electrophysiological equipment, power supply, and a workstation **55** for controlling operation of system **10**. Electrophysiological equipment of system **10** may include for example, multiple catheters, location pad **25**, body surface ECG electrodes **18**, electrode patches **38**, ablation energy generator **50**, and recorder **11**. Optionally and preferably, PIU **30** additionally includes processing capability for implementing real-time computations of location of the catheters and for performing ECG calculations.

Workstation **55** includes memory, processor unit with memory or storage with appropriate operating software stored therein, and user interface capability. Workstation **55** may provide multiple functions, optionally including (1) modeling the endocardial anatomy in three-dimensions (3D) and rendering the model or anatomical map **20** for display on a display device **27**, (2) displaying on display device **27** activation sequences (or other data) compiled from recorded electrograms **21** in representative visual indicia or imagery superimposed on the rendered anatomical map **20**, (3) displaying real-time location and orientation of the at least one catheter within the heart chamber, and (4) displaying on display device **27** sites of interest such as places where ablation energy has been applied. One commercial product embodying elements of the system **10** is available as the CARTO™ 3 System, available from Biosense Webster, Inc., 31A Technology Drive, Irvine, CA 92618.

In some examples, an irrigation module is provided for delivering irrigation fluid, such as saline solution, to the location of treatment. The irrigation module may comprise a pump and an associated fluid tank.

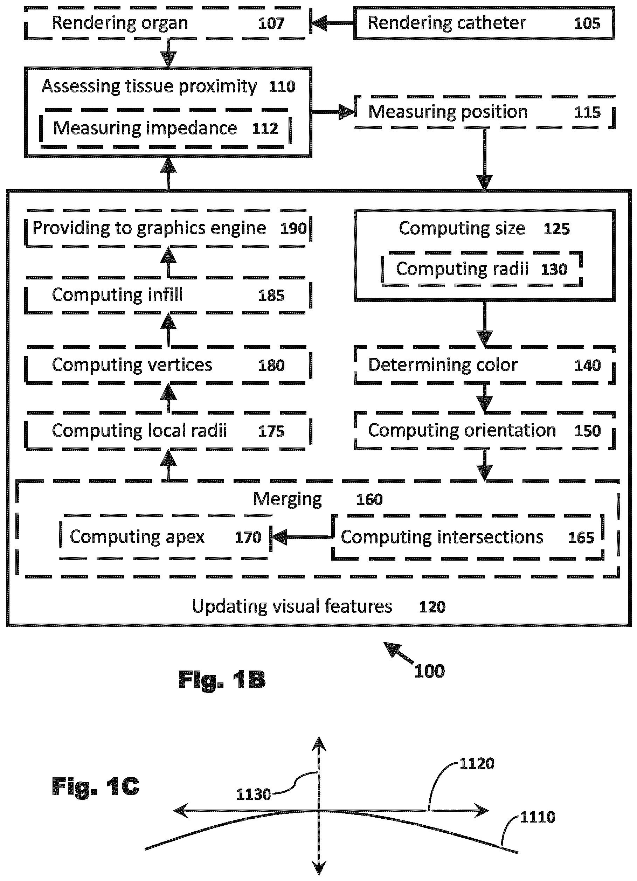

Reference is made to **FIG. 1B** showing a flowchart illustrating a computer-implemented method **100** according to embodiments of the present disclosure. The method **100** may be used for providing real-time visual feedback. That is, providing visual feedback with negligible time delay. The visual feedback may be of a contact level between a tissue wall of a luminal organ and an ablation catheter (e.g., catheter **14** illustrated in **FIG. 1A**). The catheter may include a plurality of electrodes that may be placed along a catheter distal assembly. The plurality of electrodes may form an elongated electrode array.

In some embodiments, the plurality of electrodes may form a planar electrode array. For example, the catheter distal assembly may include a single surface on which electrodes may be disposed. In some embodiments, all electrodes included in the plurality of electrodes may form a linear array. For example, a single electrode may be included in each frame element.

The method **100** may be performed while the catheter distal assembly is in the luminal organ of a patient. In some embodiments, the luminal organ may be a heart of the patient, or a portion thereof.

The method **100** may include a step **105** of rendering on a display a graphical representation of the catheter distal assembly and the plurality of electrodes thereon. In some embodiments, the method **100** may include a step **107** of rendering on the display an anatomy of the luminal organ.

The method **100** may include a step **110** of repeatedly assessing tissue proximity. In some embodiments, the step **110** of repeatedly assessing tissue proximity may include a step **112** of repeatedly measuring impedance of at least one electrode. In other words, a predefined number of impedance measurements may be performed at a predefined time period. Preferably, impedance of each of the plurality of electrodes may be repeatedly measured.

In some embodiments, the method **100** may include a step **115** of measuring a position of the at least one electrode. Preferably, position of each of the plurality of electrodes may be measured. Step **115** of measuring the position of the at least one electrode may be required, for example, if the catheter was manipulated so the at least one electrode was moved from a known position. In some embodiments, step **115** of measuring the position may be performed before the step **110** of repeatedly measuring impedance of at least one electrode.

The method **100** may include a step **120** of dynamically updating visual features. The visual features may be indicative of the measured impedance. In other words, visual features may be updated according to the measured impedance. The updating may be performed immediately upon a measurement value being received. The updating may be performed each time an impedance measurement is received. The step **120** of dynamically updating visual features may include performing steps or routines according to the exact nature of the visual features. The routines and visual features are further described hereinbelow.

The visual features may include a plurality of contact lobes. Contact lobes may be line segments having visual properties indicative of the contact level between the tissue wall and the catheter distal assembly. The contact lobes may correspond to the plurality of electrodes along the catheter distal assembly. The contact lobes may be distinct. That is, the line segments forming the contact lobes may be configured so that each contact lobe may be easily distinguished from an adjacent contact lobe by a human watching a rendering of the contact lobes, such as a physician operating the catheter. It is noted that the contact lobes may form a continuous contour, as described herein below.

A size of a contact lobe may increase with the contact level between the tissue wall and the corresponding electrode, based on the measured impedance. For example, the contact lobes may have a geometry of circular arcs. The size of the contact lobes may be a radius of the circle on which the contact lobes may be based and/or a length of the arcs.

It is noted that the size of a contact lobe may be length of a specific geometrical feature of the contact lobe. In other words, the shape of the contact lobes may be variable. For example, the contact lobes may have a geometry of ellipsoidal arcs. Their size may be a length of one of the semi-major axis or the semi-minor axis. In some embodiments, one axis may be constant. That is, if the size is the length of the semi-major axis, the semi-minor axis may be constant. If the size is the length of the semi-minor axis, the semi-major axis may be constant.

Reference is made to **FIG. 1C,** schematically illustrating transverse and tangential directions, according to embodiments of the present disclosure. A catheter distal assembly **1110** may define a tangential direction **1120** and a transverse direction **1130**. The tangential direction **1120** may be along a local orientation of the catheter distal assembly **1110**. In other words, the tangential direction **1120** may be along a line tangent to a contour of the catheter distal assembly **1110**. The transverse direction **1130** may be perpendicular to the tangential direction **1120**.

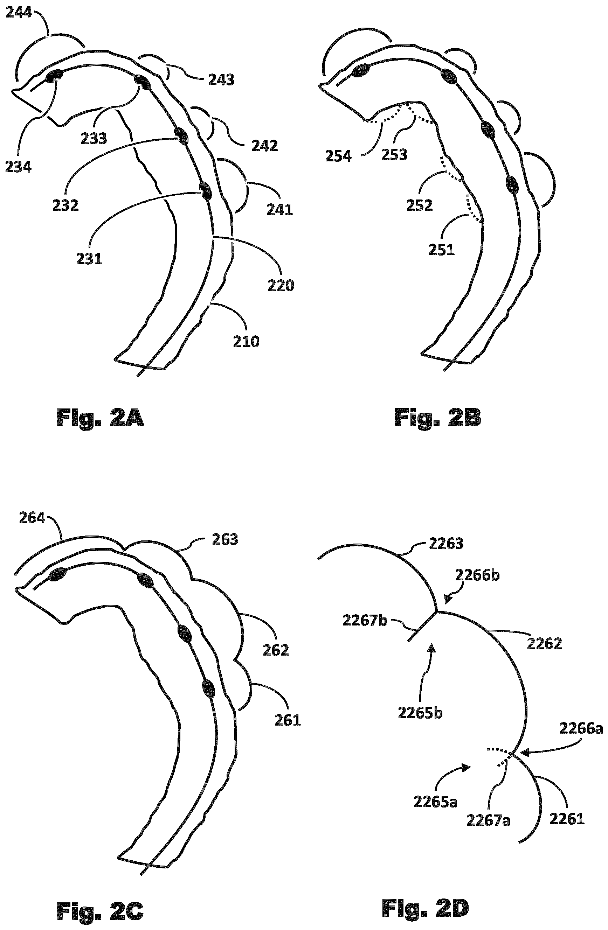

Reference is made to **FIG. 2A,** schematically illustrating exemplary contact lobes according to embodiments of the present disclosure. A catheter distal assembly **220** that may be positioned inside an intraluminal organ **210** may be rendered on a display. The catheter distal assembly **220** may include a plurality of electrodes **231, 232, 233,** and **234.** For each electrode, a corresponding contact lobe is rendered. That is, contact lobe **241** may correspond to electrode **231,** contact lobe **242** may correspond to electrode **232,** contact lobe **243** may correspond to electrode **233,** and contact lobe **244** may correspond to electrode **234.** The contact lobes **241, 242, 243,** and **244** may be overlaid on the graphical representation of the catheter distal assembly **220.**

The contact lobes **241, 242, 243,** and **244** may each be centered on a corresponding electrode. That is, the line segments forming the contact lobes **241, 242, 243,** and **244** may be configured so that each contact lobe may easily indicate the corresponding electrode by a human watching a rendering of the contact lobes, such as a physician operating the catheter. For example, the contact lobes **241, 242, 243,** and **244** may have a symmetry axis. The symmetry axis may pass through the corresponding electrode. In some embodiments, the symmetry axis may pass nearby the corresponding electrode. In some embodiments, the symmetry axis may be parallel to the transverse direction of the distal assembly **220.**

The contact lobes **241, 242, 243,** and **244** may follow a contour of the graphical representation of the catheter distal assembly **220.** In other words, for each of the contact lobes **241, 242, 243,** and **244,** an axis defining a longitudinal extent of the contact lobe may be parallel to the tangential direction the catheter distal assembly **220.** For example, the contact lobes **241, 242, 243,** and **244** may be ellipsoidal segments. In other words, the contact lobes **241, 242, 243,** and **244** may have a geometry of ellipsoidal arcs. An axis of the ellipse (i.e., the major axis or the minor axis) may be parallel to the tangential direction of the catheter distal assembly **220.**

The contact lobes **241, 242, 243,** and **244** may be positioned on one side of the catheter distal assembly **220.** The side of the catheter distal assembly **220** on which contact lobes **241, 242, 243,** and **244** may be positioned, may indicate the side of the organ **210** which the plurality of electrodes **231, 232, 233,** and **234** are in contact with. In other words, a line parallel to the transverse direction, connecting a point on a contact lobe to a corresponding electrode, may pass through the side of the organ **210** which the electrode is in contact with, but not through a side of the organ **210** which the electrode is not in contact with.

Reference is made to **FIG. 2B** schematically illustrating other exemplary contact lobes according to embodiments of the present disclosure. The embodiments illustrated in **FIG. 2B** add to the embodiments illustrated in **FIG. 2A** contact lobes **251, 251, 253,** and **254.** For brevity, reference numerals of elements already referred to in describing **FIG. 2A** are not shown in **FIG. 2B.** Contact lobe **251** corresponds to electrode **231,** contact lobe **252** corresponds to electrode **232,** contact lobe **253** corresponds to electrode **233,** and contact lobe **254** corresponds to electrode **234.** Contact lobes **251, 251, 253,** and **254** may be positioned on a side of the catheter distal assembly **220** opposite to a side on which contact lobes **241, 242, 243,** and **244** may be positioned.

In some embodiments, the contact lobes may be configured to expand asymmetrically with respect to a central axis of the catheter distal assembly, so as to predominantly extend in a transverse direction towards the tissue wall. In other words, contact lobes **241, 242, 243,** and **244** may have a greater size than contact lobes **251, 251, 253,** and **254.**

As indicated herein above, the contact lobes may form a continuous contour. In other words, at least two contact lobes may intersect or may share a common point. Preferably, in some embodiments, all contact lobes may form a continuous contour. Reference is made to **FIG. 2C,** schematically illustrating yet other exemplary contact lobes, forming a continuous contour, according to embodiments of the present disclosure. Contact lobes **261, 262, 263,** and **264** may form a continuous contour.

In yet other words, contact lobes that may form a continuous contour may be described as merged into a single area. It is noted, however, that merged contact lobes may include contact lobes forming a discontinuous contour, as described hereinbelow.

Some elements and settings illustrated in **FIG. 2C** may be similar to those illustrated in **FIG. 2A.** Therefore, for brevity, reference numerals of elements already referred to in describing **FIG. 2A** are not shown in **FIG. 2C.**

In embodiments where the contact lobes may form a continuous contour, the contact lobes may include a plurality of intersection regions. The intersection regions may distinguish between contact lobes. Reference is made to **FIG. 2D,** schematically illustrating intersection regions. Between contact lobes **2261** and **2262** an intersection region **2265a** may form. Between contact lobes **2262** and **2263** an intersection region **2265b** may form. Intersection region **2265a** may include an apex **2266a.** Intersection region **2265b** may include an apex **2266b.** An apex may be a sharp change in a direction of the continuous contour formed by contact lobes, for example, a notched saddle or a pointed dip. In another example, an apex may be a point where a parametrization of coordinates of points of the contour formed by contact lobes, as a function of some parameter, may admit a discontinuous derivative. In some embodiments, a derivative of a parametrization of any coordinate of points of the contour may have a magnitude (absolute value) greater than a predefined threshold. An apex may aid distinguishing between contact lobes.

In some embodiments, the presence of an apex may depend on measured impedance. For example, if the measured impedance of adjacent electrodes is above a predefined threshold, the contact lobes corresponding to the adjacent electrodes may form an apex at the intersection region.

It is noted that rendering an apex may not require computing derivatives. For example, intersection points between contact lobes may be computed. Contact lobes may be delimited by the intersection points. In other words, sections of the contact lobes (e.g., section **2267a,** illustrated in dashed lines) may be removed.

In some embodiments, delimitation lines may be included in intersection regions in order to aid distinguishing between contact lobes, for example, delimitation line **2267b** included in intersection region **2265b.** It is noted that the feature of delimitation line may be independent from the feature of an apex.

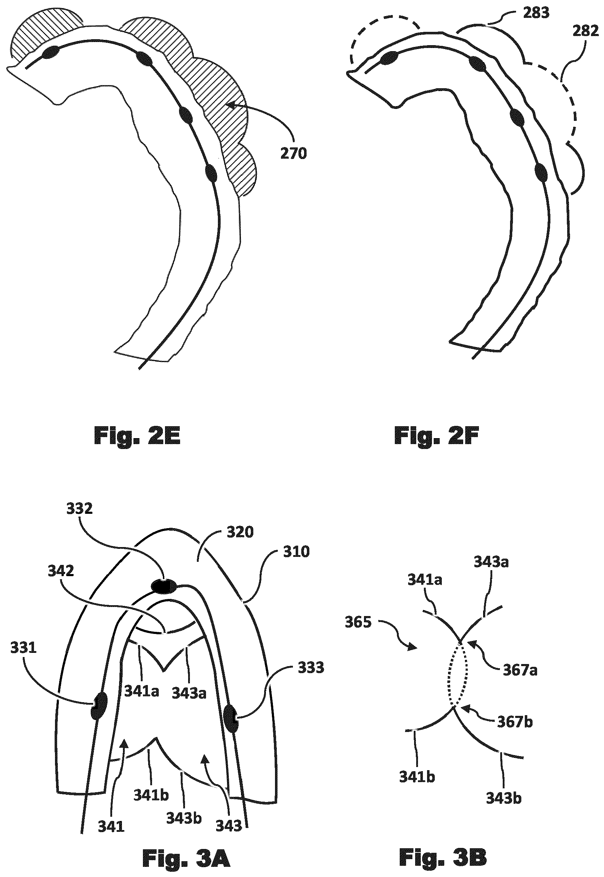

Reference is made to **FIG. 2E,** schematically illustrating yet other exemplary contact lobes according to embodiments of the present disclosure. Contact lobes may include an infill **270,** in other words, a color shading of an area region extending between a rendering of the contact lobes and rendering of the catheter distal assembly. In some embodiments, an infill region may extend between a rendering of the contact lobes and a rendering of the organ. The infill may be transparent. That is, the color shading may not conceal visual features where the infill is applied. For example, if an infill is applied on a region including a part of rendering of the organ, the structure of the part of the organ may be visible. In some embodiments, the color of the infill may be the same as a color of the contact lobes. In some embodiments, the color of the infill may be gradient. In other words, the color of the infill may gradually change. It is noted that the feature of infill may be independent from the feature of contact lobes forming a continuous contour.

Reference is made to **FIG. 2F,** schematically illustrating a variable coloring of contact lobes. In some embodiments, a color of a contact lobe may depend on a measured impedance value of a corresponding electrode. For example, contact lobe **282** may be colored by a first color (illustrated as a dashed line), whereas contact lobe **283** may be colored by a second color (illustrated as a continuous line). The coloring of the contact lobes **282** and **283** may indicate a contact level being above or below a certain threshold. In other words, a first color may indicate the contact level being lower than a minimum threshold, and a second color may indicate the contact level being greater than the minimum threshold.

Some elements and settings illustrated in **FIGS. 2E-2F** may be similar to those illustrated in **FIG. 2C.** Therefore, for brevity, reference numerals of elements already referred to in describing **FIG. 2C** are not shown in **FIGS. 2E-2F.**

Reference is made to **FIGS. 3A-3B,** schematically illustrating merging of non-adjacent contact lobes, according to embodiments of the present disclosure. A catheter distal assembly **320** that may be positioned inside an intraluminal organ **310** may be rendered on a display. The catheter distal assembly **320** may include a plurality of electrodes **331, 332,** and **333.** Contact lobes corresponding to electrodes **331** and **333** may be non-adjacent. That is, the corresponding electrodes **331** and **333** may not be nearest neighbors in the array of electrodes, or may not be adjacent in the linear array of electrodes. For each electrode, a corresponding contact lobe is rendered. That is, contact lobe **341** may correspond to electrode **331,** contact lobe **342** may correspond to electrode **332,** and contact lobe **343** may correspond to electrode **233.** The contact lobes may be overlaid on the graphical representation of the catheter distal assembly **320.**

Contact lobes corresponding to electrodes **331 333** may be merged. It is noted that merging of non-adjacent contact lobes may result in a discontinuous contour. This may contrast merging of adjacent contact lobes described herein above. For example, the contour of contact lobe **341** may be split into two discontinuous segments **341a** and **341b,** and the contour of contact lobe **343** may be split into two discontinuous segments **343a** and **343b.** An intersection region **365** may include apex **367a** formed between segments **341a** and **343a,** and apex **367b** formed between segments **341b** and **343b.**

Merging of non-adjacent contact lobes may be required, for example, if the catheter distal assembly **320** is sharply bent, e.g., the catheter distal assembly **320** may have a "U-turn".

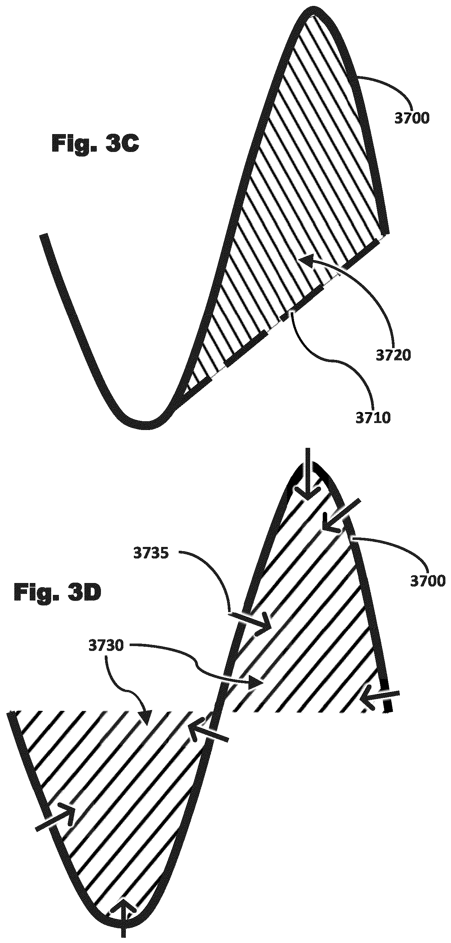

It is noted that in **FIG. 2A,** the contact lobes are "outside" the catheter distal assembly, while in **FIG. 3A,** they are "inside" the catheter distal assembly. In other words, in **FIG. 2A,** the contact lobes are positioned in an outer side of the catheter distal assembly, while in **FIG. 3A,** the contact lobes are positioned in an inner side of the catheter distal assembly. Reference is made to **FIGS. 3C-3E,** schematically illustrating an inner side according to embodiments of the present disclosure. An "inner side" and an "outer side" of a segment of the catheter distal assembly **3700** can be defined in two ways. An outer side may be defined as any area that is not included in the inner side.

Reference is made to **FIG. 3C** schematically illustrating an inner side according to a first definition. According to the first definition, the inner side may be an area **3720** delimited by a chord line **3710** between two points on the segment, and the catheter distal assembly **3700.** Area **3720** is marked with down-going diagonal stripes.

Reference is made to **FIG. 3D** schematically illustrating an inner side according to a second definition. According to the second definition, the inner side **3730** may be where displacement, along the transverse direction, forms an acute angle with a local curvature vector, such as local curvature vector **3735.** Area **3730** is marked with up-going diagonal stripes.

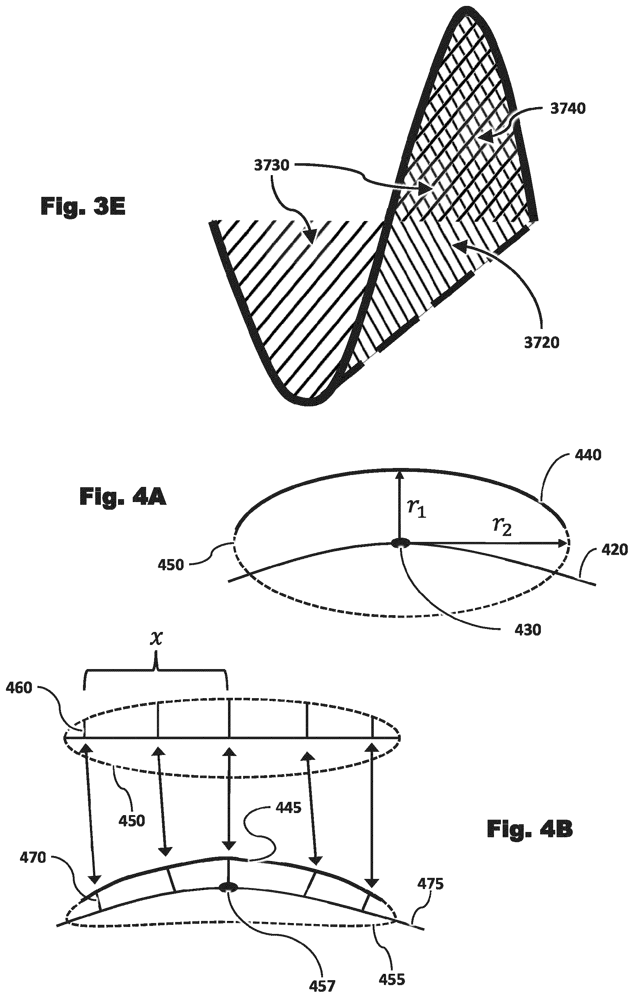

It is noted that the two definitions hereinabove (of an inner side) may not necessarily be mutually exclusive. As illustrated in **FIG. 3E,** an area **3740** may be included in the inner side according to both the first definition and the second definition. In other words, area **3740** may be included in area **3720** and in area **3730.** Area **3740** is marked with crossing diagonal lines. In other words, area **3740** is marked with both down-going diagonal stripes (marking area **3720)** and up-going diagonal stripes (marking area **3730).**

Depending on the segment, either of these definitions can be used to define the inner and outer sides of the catheter distal assembly. In some embodiments, methods disclosed herein (e.g., method 100) may include selecting a definition of an inner side, for at least one segment of the catheter distal assembly. In some embodiments, selecting a definition of an inner side may be performed dynamically, i.e., according to a computation performed in real-time, based on measured positions of electrodes included in the catheter distal assembly.

In some embodiments, contact lobes may be positioned exclusively outside the catheter distal assembly. The contact lobes may not necessarily indicate the side of the organ in contact with the electrodes. In embodiments where contact lobes expand asymmetrically relative to the catheter's central axis, they may expand towards the outer side. Placing the contact lobes outside, or expanding them asymmetrically outward, may allow for larger contact lobes, improving the visualization of the contact level.

Reference is made to **FIG. 4A,** schematically illustrating transverse and tangential radii, according to embodiments of the present disclosure. A catheter distal assembly **420** may have an electrode **430.** The catheter distal assembly **420,** the electrode **430,** and a contact lobe **440** may be rendered on a display. The contact lobe **440** may be an ellipsoidal segment. In other words, contact lobe **440** may have a geometry of an ellipsoidal arc. In other words, contact lobe **440** may be a line segment of an ellipse **450.** The ellipse **450** may not be rendered on the display. The contact lobe **440** may be oriented along a tangent direction of the catheter distal assembly **420.** In other words, the ellipse **450** may have two axes: a major axis and a minor axis. One axis (e.g., the major axis) may be parallel to the tangential direction. The other axis (e.g., the minor axis) may be parallel to the transverse direction. A half of the axis parallel to the tangential direction may be referred to as a "tangential radius". The tangential radius may extend along the tangential direction. The tangential radius may be of length $r_1$. A half of the axis parallel to the transverse direction may be referred to as a "transverse radius". The transverse radius may extend along the transverse direction. The transverse radius may be of length $r_2$. Similarly, the axis parallel to the tangential direction may be referred to as a "tangential diameter", and the axis parallel to the transversal direction may be referred to as a "transversal diameter".

In some embodiments, the tangential radius may be constant. In some embodiments, the tangential radius may be based on a spacing between the electrodes of the electrode array. In some embodiments, the tangential radius may be equal to half a sum of distances to adjacent electrodes along the catheter distal assembly. In other words, the tangential radius may be equal to an average separation of a corresponding electrode to nearby electrodes, where the separation may be measured along the catheter distal assembly.

Reference is made to **FIG. 4B,** schematically illustrating bending of contact lobes, according to embodiments of the present disclosure. In some embodiments, where the contact lobes may include ellipsoidal segments, some of the ellipsoidal segments may be bent according to a curviness of the catheter distal assembly **475.** A contact lobe **445** may have a shape of a bent ellipsoidal segment. Each point of the contact lobe **445** may have a distance **470** from the catheter distal assembly **475** that may be equal to a distance **460** between a tangential radius of a regular (unbent) ellipse **450** and a corresponding point on the rim of the ellipse. In other words, the tangential diameter of the ellipse **450** may be bent according to the curviness of the catheter distal assembly **475,** while a local half-thickness of the ellipse **450** may be kept. In yet other words, a point on the ellipse **450** may have a distance of $r_1 \sqrt{\left(x/r_2\right)^2 - 1}$ from the tangent diameter, where $x$ may denote a distance along the tangent diameter from the center of the ellipse **450**. A point on a bent ellipse **455** may have a distance of $r_1 \sqrt{\left(y/r_2\right)^2 - 1}$ from the catheter distal assembly **475,** where y may denote a distance along the catheter distal assembly from a center point. In some embodiments, the center point may be an electrode **457.** In other words, in some embodiments, a point on a bent ellipse **455** may have a distance of $r_1 \sqrt{\left(y/r_2\right)^2 - 1}$ from the catheter distal assembly **475,** where y may denote a distance along the catheter distal assembly from a corresponding electrode **457.** This distance may be referred to as a "local radius".

Reference is made to **FIG. 1A.** The step **120** of dynamically updating visual features may include a step **125** of computing a size of the contact lobes. In some embodiments, where contact lobes may include ellipsoidal segments, the step **125** of computing a size of the contact lobes may include a step **130** of computing the radius of the ellipsoidal segments (e.g., tangential radius and transverse radius). In embodiments where a transverse radius may be computed, the transverse radius may be variable. The transverse radius may be based on the contact level between the tissue wall and an electrode. As indicated hereinabove, the transverse radius may extend in a direction transverse to a local orientation of the catheter distal assembly around the (corresponding) electrode.

In some embodiments, the variable transverse radius may vary within a radius adjustment range bounded by a minimum radius threshold value and a maximum radius threshold value. In other words, the variable transverse radius may have a minimum value and a maximum value. In some embodiments, the minimum radius threshold may correspond to a zero-contact level. In other words, the minimum radius threshold may correspond to a state of no-contact level between the tissue wall and the electrode.

In some embodiments, the step **120** of dynamically updating visual features may include a step **140** of determining a color of the contact lobes (e.g., according to a contact level, as described hereinabove).

In some embodiments, the step **120** of dynamically updating visual features may include a step **150** of computing orientation of the contact lobes. For example, as indicated hereinabove, an orientation of a contact lobe may be along a tangential direction. It is noted, however, that orientation of contact lobes may not be limited to the transverse direction. The step **150** of computing orientations of contact lobes may include, for example, computing line-parameters of a lines defining a longitudinal extent of the contact lobes.

In some embodiments, the step **120** of dynamically updating visual features may include a step **160** of merging contact lobes. In some embodiments, the step **160** of merging contact lobes may include a step **165** of computing intersection points between contact lobes.

In some embodiments, the step **160** of merging contact lobes may include a step **170** of computing an apex between contact lobes. The step **170** of computing an apex may include determining whether an apex may be included. In some embodiments, the step **170** of computing an apex may include comparing a transverse radius to an adjacent transverse radius of an adjacent electrode. If a difference between the transverse radius and the adjacent transverse radius is lower than a predefined comparison threshold, a local transverse radius at an intersection point may be lower than the transverse radius by a predefined dip threshold. In other words, an intersection point may be closer to the catheter distal assembly than the transverse radius by at least a predefined threshold.

In some embodiments, the step **120** of dynamically updating visual features may include a step **175** of computing local transverse radii. The step **175** of computing local transverse radii may include computing for each contact lobe, a plurality of local transverse radius at a corresponding plurality of positions according to a tangential distance from the electrode. In other words, distances from the catheter distal assembly, or from a reference axis (e.g., an axis defining a longitudinal extent or an axis of an ellipse), may be computed. In some embodiments, a local radius is computed for distance increments being 0.5 millimeters. The distance increments may be distance increments along the catheter distal assembly.

In some embodiments, the step **120** of dynamically updating visual features may include a step **180** of computing vertices. In other words, coordinates of points of the contact lobes may be computed. In some embodiments, the vertices may be vertices of polygons approximating the exact shape of the contact lobes. Polygons approximating the exact shape of the contact lobes may be required, for example, if a computer implementing the method **100** may require curves to be approximated by a plurality of straight lines.

In some embodiments, the step **120** of dynamically updating visual features may include a step **185** of computing an infill. The step **185** of computing an infill may include determining whether an infill is to be included. If an infill is included, the step **185** of computing an infill may include computing a desired color of the infill. The step **185** of computing an infill may include computing a transparency of the infill, e.g., an alpha channel of an RGBA color format.

In some embodiments, the step **120** of dynamically updating visual features may include a step **190** of providing computation results to a graphics engine, in order to render the contact lobes. In some embodiments, some of the computations may be performed by the graphics engine, for example the step **180** of computing vertices.

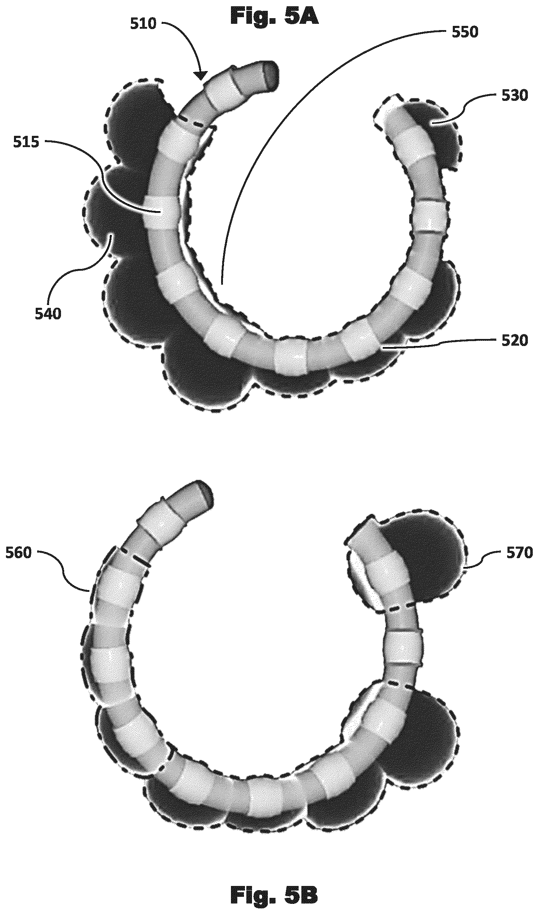

Reference is made to **FIG. 5A,** showing an exemplary screenshot of visual output of methods and systems according to embodiments of the present disclosure. A catheter distal assembly **510** comprising a plurality of electrodes (such as electrode **515**) is rendered along with a plurality of contact lobes such as contact lobes **520, 530, 540,** and **550.** The contact lobes are rendered with dashed lines. The contact lobes expand asymmetrically with respect to a central axis of the catheter distal assembly, so as to predominantly extend in a transverse direction towards the tissue wall. The contact lobes **520, 530, 540,** and **550** are ellipsoidal segments. Some of the contact lobes form a single contour. The contact lobes include an infill.

Reference is made to **FIG. 5B,** showing another exemplary screenshot of visual output of methods and systems according to embodiments of the present disclosure. Contact lobes are rendered with dashed lines. Contact lobe **560** has one color, whereas contact lobe **570** has a second color. The different colors are shown as different types of dashes. Contact lobe **560** is an ellipsoidal segment, whereas contact lobe **570** is not. Contact lobe **560** includes an infill, whereas contact lobe **570** does not. Contact lobe **560** has a minimum transverse radius. Some of the contact **lobes** form a single contour.

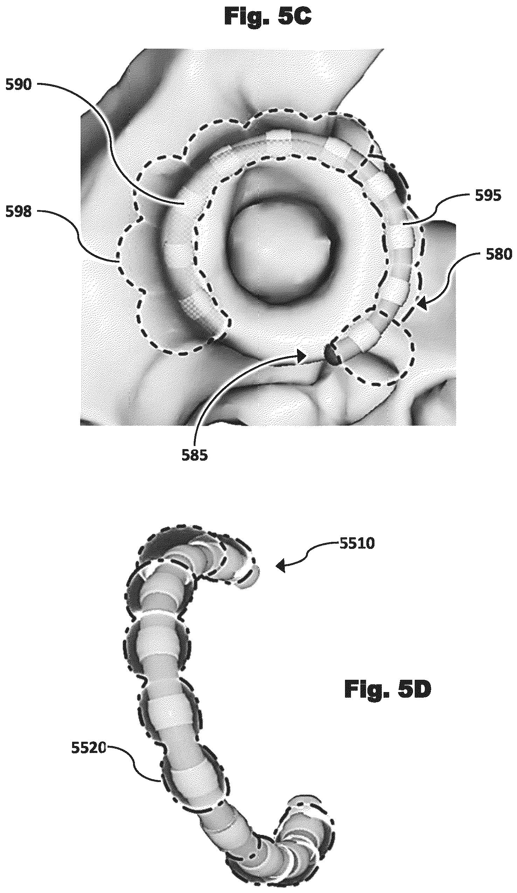

Reference is made to **FIG. 5C,** showing yet another exemplary screenshot of visual output of methods and systems according to embodiments of the present disclosure. A catheter distal assembly **585** comprising a plurality of electrodes (such as electrode **590**) is rendered along with a plurality of contact lobes such as contact lobes **595** and **598.** Contact lobes are rendered with dashed lines. An organ **580** is also rendered. The contact lobes expand asymmetrically with respect to a central axis of the catheter distal assembly, so as to predominantly extend in a transverse direction towards the tissue wall. Contact lobe **595** has a minimum transverse radius. Contact lobe **595** has one color, whereas contact lobe **598** has a second color. The different colors are shown as different types of dashes. All of the contact lobes form a single contour.

Reference is made to **FIG. 5D,** showing yet another exemplary screenshot of visual output of methods and systems according to embodiments of the present disclosure. A catheter distal assembly **5510** comprising a plurality of electrodes is rendered along with a plurality of contact lobes such as contact lobe **5520.** The viewing position of the catheter distal assembly **5510** in **FIG. 5D** is from the side, whereas the viewing position of catheter distal assemblies in **FIGS. 5A-5C** is from the top. It is noted that the contact lobes may be three-dimensional objects, where each contact lobe may include a plurality of line segments (e.g., ellipsoidal segments) that may be rendered on a display.

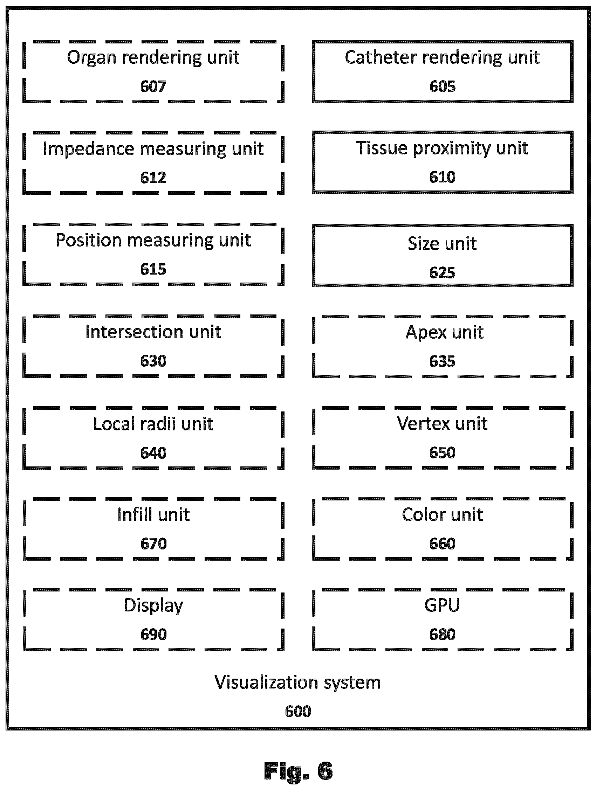

Reference is made to **FIG. 6** which shows a block diagram schematically illustrating a system according to embodiments of the present disclosure. A visualization system **600** may be configured to implement methods according to the present disclosure (e.g., method **100** schematically illustrated in **FIG. 1B**) in order to provide, in real-time, visual feedback of a contact level between a tissue wall of a luminal organ and an ablation catheter. The system **600** may be, for example, a computer system.

The visualization system **600** may include a catheter rendering unit **605,** in order to render the catheter distal assembly. The visualization system **600** may include an organ rendering unit **607,** in order to render the organ in which the catheter distal assembly is positioned. The visualization system **600** may include a tissue proximity unit **610,** in order to asses proximity (contact level) of electrodes to the tissue wall. The visualization system **600** may include an impedance measuring unit **612,** in order to measure impedance of at least one electrode included in the catheter distal assembly. The visualization system **600** may include a position measuring unit **615,** in order to measure a position of the catheter distal assembly. The visualization system **600** may include a size unit **625,** in order to compute the size of contact lobes (e.g., transverse radius). The visualization system **600** may include an intersection unit **630,** in order to compute intersection points between contact lobes. The visualization system **600** may include an apex unit **635,** in order to determine whether an apex may be required at an intersection region between contact lobes, and compute parameters of the apex. The visualization system **600** may include a local radii unit **640,** in order to compute local radii (a plurality of local radius) of points of contact lobes. The visualization system **600** may include a vertex unit **650,** in order to compute coordinates of points of the contact lobes. The visualization system **600** may include a color unit **660,** in order to determine a color of the contact lobes. The visualization system **600** may include an infill unit **670,** in order to determine whether an infill is included in a contact lobe. The visualization system **600** may include a GPU **680,** in order to efficiently perform rendering computations. The visualization system **600** may include a display **690,** in order to display the visual feedback.

## SUMMARY

Following is a non-exclusive list of some exemplary examples of the disclosure. The present disclosure also includes examples which include fewer than all the features in an example, and examples using features from multiple examples, even if not listed below.

**Example 1:** A computer-implemented method of providing real-time visual feedback of a contact level between a tissue wall of a luminal organ and an ablation catheter. The ablation catheter comprising a plurality of electrodes forming an elongated electrode array placed along a catheter distal assembly. The method comprising, while the catheter distal assembly is in the luminal organ of a patient, rendering on a display a graphical representation of the catheter distal assembly and the plurality of electrodes thereon. The method comprising, while the catheter distal assembly is in the luminal organ of a patient, for each electrode of the plurality of electrodes, repeatedly assessing tissue proximity of each of the plurality of electrodes. Further, for each electrode of the plurality of electrodes, the method comprising dynamically updating visual features indicative of the tissue proximity. The visual features comprising contact lobes, wherein each contact lobe is centered on a corresponding electrode and overlaid on said graphical representation of the catheter distal assembly, and a size of said contact lobe increases with the contact level between said tissue wall and said corresponding electrode, based on the tissue proximity.

**Example 2:** The computer-implemented method according to example 1, wherein assessing tissue proximity comprises measuring impedance of at least one electrode.

**Example 3:** The computer-implemented method according to any one of examples 1 to 2, wherein when the tissue proximity of adjacent electrodes is above a predefined threshold, the contact lobes corresponding to said adjacent electrodes form an apex at an intersection region.

**Example 4:** The computer-implemented method according to any one of examples 1 to 3, wherein said contact lobes are configured to expand asymmetrically with respect to a central axis of the catheter, so as to predominantly extend in a transverse direction towards the tissue wall.

**Example 5:** The computer-implemented method according to any one of examples 3 to 4, comprising merging overlapping contact lobes, corresponding to distinct electrodes, into a single area.

**Example 6:** The computer-implemented method according to any one of examples 1 to 5, wherein said contact lobes comprise ellipsoidal segments.

**Example 7:** The computer-implemented method according to example 6, comprising, for each electrode, computing a variable transverse radius of said ellipsoidal segments, said variable transverse radius being based on the contact level between the tissue wall and said electrode and extending in a direction transverse to a local orientation of the catheter around said electrode.

**Example 8:** The computer-implemented method according to example 7, comprising comparing a transverse radius to an adjacent transverse radius of an adjacent electrode. Wherein, when a difference between the transverse radius and the adjacent transverse radius is lower than a predefined comparison threshold, a local transverse radius at an intersection point is lower than the transverse radius by a predefined dip threshold.

**Example 9:** The computer-implemented method according to any one of examples 7 to 8, wherein said variable transverse radius varies within a radius adjustment range bounded by a minimum radius threshold value and a maximum radius threshold value.

**Example 10:** The computer-implemented method according to example 9, wherein said minimum radius threshold corresponds a zero-contact level.

**Example 11:** The computer-implemented method according to any one of examples 6 to 10, wherein said ellipsoidal segments include a tangential radius extending in a direction of the local orientation of said catheter.

**Example 12:** The computer-implemented method according to example 11, wherein said tangential radius is constant and based on a spacing between the electrodes of the electrode array.

**Example 13:** The computer-implemented method according to any one of examples 11 to 12, wherein the tangential radius equals half a sum of distances to adjacent electrodes along the catheter.

**Example 14:** The computer-implemented method according to any one of examples 11 to 13, wherein the local orientation of the catheter around a given electrode is defined according to a line tangent to said catheter distal assembly at a location of the given electrode.

**Example 15:** The computer-implemented method according to any one of examples 3 to 14, comprising merging overlapping contact lobes, corresponding to distinct electrodes, into a single area.

**Example 16:** The computer-implemented method according to any one of examples 1 to 15, comprising coloring the contact lobes using a transparent infill.

**Example 17:** The computer-implemented method according to example 16, wherein a first color indicates the contact level being lower than a minimum threshold, and a second color indicates the contact level being greater than the minimum threshold.

**Example 18:** The computer-implemented method according to any one of examples 6 to 17, wherein said ellipsoidal segments are bent according to a curviness of the catheter.

**Example 19:** The computer-implemented method according to any one of examples 1 to 18, comprising computing for each contact lobe, a plurality of local transverse radius at a corresponding plurality of positions at according to a tangential distance from the electrode.

**Example 20:** The computer-implemented method according to example 19, wherein said local radius is computed for distance increments being 0.5 millimeter.

**Example 21:** The computer-implemented method according to any one of examples 1 to 20, wherein the luminal organ is a heart of the patient.

**Example 22:** The computer-implemented method according to any one of examples 1 to 21, wherein all electrodes included in the plurality of electrodes forms a linear array.

**Example 23:** The computer-implemented method according to any one of examples 1 to 22, comprising rendering on the display an anatomy of the luminal organ.

**Example 24:** A graphical user interface (GUI) for providing real-time visual feedback of contact level between a tissue wall of a luminal organ and an ablation catheter. The ablation catheter comprising a plurality of electrodes forming an elongated electrode array placed along a catheter distal assembly. The GUI being executable by a computer to, while the catheter distal assembly is in the luminal organ of a patient, rendering on a display a graphical representation of the catheter distal assembly and the plurality of electrodes thereon. The GUI being executable by a computer to, while the catheter distal assembly is in the luminal organ of a patient, for each electrode of the plurality of electrodes, repeatedly receive tissue proximity values of each of the plurality of electrodes. Further, for each electrode of the plurality of electrodes, the GUI being executable by a computer to dynamically update visual features indicative of a tissue proximity value, responsive to a change detected in the tissue proximity value. The visual features comprising contact lobes, wherein each contact lobe is centered on a corresponding electrode and overlaid on said graphical representation of the catheter distal assembly, and a size of said contact lobe increases with the contact level between said tissue wall and said corresponding electrode, based on the tissue proximity value.

**Example 25:** The GUI according to example 24, wherein the tissue proximity values correspond measured impedance values of the plurality of electrodes.

**Example 26:** The GUI according to any one of examples 24 to 25, wherein when the measured impedance of adjacent electrodes is above a predefined threshold, the contact lobes corresponding to said adjacent electrodes form an apex at an intersection region.

**Example 27:** The GUI according to any one of examples 24 to 26, wherein said contact lobes are configured to expand asymmetrically with respect to a central axis of the catheter, so as to predominantly extend in a transverse direction towards the tissue wall.

**Example 28:** The GUI according to any one of examples 26 to 27, comprising merging overlapping contact lobes, corresponding to distinct electrodes, into a single area.

**Example 29:** The GUI according to any one of examples 24 to 28, wherein said contact lobes comprise ellipsoidal segments.

**Example 30:** The GUI according to example 29, comprising, for each electrode, computing a variable transverse radius of said ellipsoidal segments, said variable transverse radius being based on the contact level between the tissue wall and said electrode and extending in a direction transverse to a local orientation of the catheter around said electrode.

**Example 31:** The GUI according to example 30, comprising comparing a transverse radius to an adjacent transverse radius of an adjacent electrode. Wherein, when a difference between the transverse radius and the adjacent transverse radius is lower than a predefined comparison threshold, a local transverse radius at an intersection point is lower than the transverse radius by a predefined dip threshold.

**Example 32:** The GUI according to any one of examples 30 to 31, wherein said variable transverse radius varies within a radius adjustment range bounded by a minimum radius threshold value and a maximum radius threshold value.

**Example 33:** The GUI according to example 32, wherein said minimum radius threshold corresponds a zero-contact level.

**Example 34:** The GUI according to any one of examples 29 to 33, wherein said ellipsoidal segments include a tangential radius extending in a direction of the local orientation of said catheter.

**Example 35:** The GUI according to example 34, wherein said tangential radius is constant and based on a spacing between the electrodes of the electrode array.

**Example 36:** The GUI according to any one of examples 34 to 35, wherein the tangential radius equals half a sum of distances to adjacent electrodes along the catheter.

**Example 37:** The GUI according to any one of examples 34 to 36, wherein the local orientation of the catheter around a given electrode is defined according to a line tangent to said catheter distal assembly at a location of the given electrode.

**Example 38:** The GUI according to any one of examples 26 to 37, comprising merging overlapping contact lobes, corresponding to distinct electrodes, into a single area.

**Example 39:** The GUI according to any one of examples 24 to 38, comprising coloring the contact lobes using a transparent infill.

**Example 40:** The GUI according to example 39, wherein a first color indicates the contact level being lower than a minimum threshold, and a second color indicates the contact level being greater than the minimum threshold.

**Example 41:** The GUI according to any one of examples 29 to 40, wherein said ellipsoidal segments are bent according to a curviness of the catheter.

**Example 42:** The GUI according to any one of examples 24 to 41, comprising computing for each contact lobe, a plurality of local transverse radius at a corresponding plurality of positions at according to a tangential distance from the electrode.

**Example 43:** The GUI according to example 42, wherein said local radius is computed for distance increments being 0.5 millimeter.

**Example 44:** The GUI according to any one of examples 24 to 43, wherein the luminal organ is a heart of the patient.

**Example 45:** The GUI according to any one of examples 24 to 44, wherein all electrodes included in the plurality of electrodes forms a linear array.

**Example 46:** The GUI according to any one of examples 24 to 45, comprising rendering on the display an anatomy of the luminal organ.

**Example 47:** A computer system comprising at least one processing circuitry, configured to execute a method of providing real-time visual feedback of a contact level between a tissue wall of a luminal organ and an ablation catheter. The ablation catheter comprising a plurality of electrodes forming an elongated electrode array placed along a catheter distal assembly. The method comprising, while the catheter distal assembly is in the luminal organ of a patient, rendering on a display a graphical representation of the catheter distal assembly and the plurality of electrodes thereon. The method comprising, while the catheter distal assembly is in the luminal organ of a patient, for each electrode of the plurality of electrodes, repeatedly assessing tissue proximity of each of the plurality of electrodes. Further, for each electrode of the plurality of electrodes, the method comprising dynamically updating visual features indicative of the tissue proximity. The visual features comprising contact lobes, wherein each contact lobe is centered on a corresponding electrode and overlaid on said graphical representation of the catheter distal assembly, and a size of said contact lobe increases with the contact level between said tissue wall and said corresponding electrode, based on the tissue proximity.

**Example 48:** The computer system according to example 47, wherein assessing tissue proximity comprises measuring impedance of at least one electrode.

**Example 49:** The computer system according to any one of example 47 to 48, wherein when the tissue proximity of adjacent electrodes is above a predefined threshold, the contact lobes corresponding to said adjacent electrodes form an apex at an intersection region.

**Example 50:** The computer system according to any one of examples 47 to 49, wherein said contact lobes are configured to expand asymmetrically with respect to a central axis of the catheter, so as to predominantly extend in a transverse direction towards the tissue wall.

**Example 51:** The computer system according to any one of examples 49 to 50, comprising merging overlapping contact lobes, corresponding to distinct electrodes, into a single area.

**Example 52:** The computer system according to any one of examples 47 to 51, wherein said contact lobes comprise ellipsoidal segments.

**Example 53:** The computer system according to example 52, comprising, for each electrode, computing a variable transverse radius of said ellipsoidal segments, said variable transverse radius being based on the contact level between the tissue wall and said electrode and extending in a direction transverse to a local orientation of the catheter around said electrode.

**Example 54:** The computer system according to example 53, comprising comparing a transverse radius to an adjacent transverse radius of an adjacent electrode. Wherein, when a difference between the transverse radius and the adjacent transverse radius is lower than a predefined comparison threshold, a local transverse radius at an intersection point is lower than the transverse radius by a predefined dip threshold.

**Example 55:** The computer system according to any one of examples 53 to 54, wherein said variable transverse radius varies within a radius adjustment range bounded by a minimum radius threshold value and a maximum radius threshold value.

**Example 56:** The computer system according to example 55, wherein said minimum radius threshold corresponds a zero-contact level.

**Example 57:** The computer system according to any one of examples 52 to 55, wherein said ellipsoidal segments include a tangential radius extending in a direction of the local orientation of said catheter.

**Example 58:** The computer system according to example 57, wherein said tangential radius is constant and based on a spacing between the electrodes of the electrode array.

**Example 59:** The computer system according to any one of examples 57 to 58, wherein the tangential radius equals half a sum of distances to adjacent electrodes along the catheter.

**Example 60:** The computer system according to any one of examples 57 to 59, wherein the local orientation of the catheter around a given electrode is defined according to a line tangent to said catheter distal assembly at a location of the given electrode.

**Example 61:** The computer system according to any one of examples 49 to 60, comprising merging overlapping contact lobes, corresponding to distinct electrodes, into a single area.

**Example 62:** The computer system according to any one of examples 47 to 61, comprising coloring the contact lobes using a transparent infill.

**Example 63:** The computer system according to example 62, wherein a first color indicates the contact level being lower than a minimum threshold, and a second color indicates the contact level being greater than the minimum threshold.

**Example 64:** The computer system according to any one of examples 62 to 63, wherein said ellipsoidal segments are bent according to a curviness of the catheter.

**Example 65:** The computer system according to any one of examples 47 to 64, comprising computing for each contact lobe, a plurality of local transverse radius at a corresponding plurality of positions at according to a tangential distance from the electrode.

**Example 66:** The computer system according to example 65, wherein said local radius is computed for distance increments being 0.5 millimeter.

**Example 67:** The computer system according to any one of examples 47 to 66, wherein the luminal organ is a heart of the patient.

**Example 68:** The computer system according to any one of examples 47 to 67, wherein all electrodes included in the plurality of electrodes forms a linear array.

**Example 69:** The computer system according to any one of examples 47 to 68, comprising rendering on the display an anatomy of the luminal organ.

**Example 70:** A non-transitory computer readable storage medium tangibly embodying a program of instructions that, when executed by a computer, cause the computer to perform a method according to any one of examples 1 to 23.

**Example 71:** A non-transitory computer readable storage medium tangibly embodying a program of instructions that, when executed by a computer, cause the computer to execute a GUI according to any one of examples 24 to 46.

Those skilled in the art to which the present disclosure pertains, can appreciate that while the present disclosure has been described in terms of preferred examples, the concept upon which this disclosure is based may readily be utilized as a basis for the designing of other structures, systems, and processes for carrying out the several purposes of the present disclosure.

Also, it is to be understood that the phraseology and terminology employed herein are for the purpose of description and should not be regarded as limiting. It should be noted that the words "comprising", "including" and "having" as used throughout the appended claims are to be interpreted to mean "including but not limited to". The indefinite articles "a" and "an," as used herein in the specification and in the claims, unless clearly indicated to the contrary, should be understood to mean "at least one." The phrase "and/or," as used herein in the specification and in the claims, should be understood to mean "either or both" of the elements so conjoined, i.e., elements that are conjunctively present in some cases, and disjunctively present in other cases. The term "each" may not be exclusively understood as referring to each and every, and, when technically relevant, may also refer to "at least some".

All patents and patent applications mentioned in this specification are herein incorporated in their entirety by reference into the specification, to the same extent as if each individual patent or patent application was specifically and individually indicated to be incorporated herein by reference. In addition, citation or identification of any reference in this application shall not be construed as an admission that such reference is available as prior art to the present disclosure.

It is important, therefore, that the scope of the present disclosure is not construed as being limited by the illustrative examples set forth herein. Other variations are possible within the scope of the present disclosure as defined in the appended claims. Other combinations and sub-combinations of features, functions, elements and/or properties, may be claimed through amendment of the present claims or presentation of new claims in this or a related application. Such amended or new claims, whether they are directed to different combinations or directed to the same combinations, whether different, broader, narrower, or equal in scope to the original claims, are also regarded as included within the subject matter of the present description.

**Claims**

1. A computer-implemented method (100) of providing real-time visual feedback of a contact level between a tissue wall of a luminal organ and an ablation catheter comprising a plurality of electrodes forming an elongated electrode array placed along a catheter distal assembly (510), the method comprising:
   while the catheter distal assembly is in the luminal organ of a patient:

   (a) rendering on a display (105) a graphical representation of the catheter distal assembly and the plurality of electrodes thereon;
   (b) for each electrode of the plurality of electrodes:

   i. repeatedly assessing tissue proximity (110) of each of the plurality of electrodes; and

ii. dynamically updating visual features indicative of the tissue proximity (120), said visual features comprising contact lobes (520, 530, 540), wherein each contact lobe is centered on a corresponding electrode (515) and overlaid on said graphical representation of the catheter distal assembly;

a size of said contact lobe increases with the contact level between said tissue wall and said corresponding electrode, based on the tissue proximity.

2. The computer-implemented method according to claim 1, wherein assessing tissue proximity comprises measuring impedance (112) of at least one electrode.

3. The computer-implemented method according to any one of claims 1 to 2, wherein when the tissue proximity of adjacent electrodes is above a predefined threshold, the contact lobes corresponding to said adjacent electrodes form an apex at an intersection region.

4. The computer-implemented method according to any one of claims 1 to 3, wherein said contact lobes are configured to expand asymmetrically with respect to a central axis of the catheter, so as to predominantly extend in a transverse direction towards the tissue wall.

5. The computer-implemented method according to any one of claims 1 to 4, wherein said contact lobes comprise ellipsoidal segments (440); and, the method comprising, for each electrode, computing a variable transverse radius of said ellipsoidal segments (130), said variable transverse radius being based on the contact level between the tissue wall and said electrode and extending in a direction transverse to a local orientation of the catheter around said electrode.

6. A graphical user interface (GUI) for providing real-time visual feedback of contact level between a tissue wall of a luminal organ and an ablation catheter comprising a plurality of electrodes forming an elongated electrode array placed along a catheter distal assembly (510), the GUI being executable by a computer to:

while the catheter distal assembly is in the luminal organ of a patient:

(a) render, on a display, a graphical representation of the catheter distal assembly and the plurality of electrodes thereon;
(b) for each electrode of the plurality of electrodes:

i. repeatedly receive tissue proximity values of each of the plurality of electrodes; and
ii. responsive to a change detected in a tissue proximity value, dynamically update visual features indicative of the tissue proximity, said visual features comprising contact lobes (520, 530, 540), wherein

each contact lobe is centered on a corresponding electrode and overlaid on said graphical representation of the catheter distal assembly; and
a size of said contact lobe increases with the contact level between said tissue wall and said corresponding electrode, based on the tissue proximity.

7. The GUI according to claim 6, wherein the tissue proximity values correspond measured impedance values of the plurality of electrodes.

8. The GUI according to any one of claims 6 to 7, wherein when the tissue proximity values of adjacent electrodes is above a predefined threshold, the contact lobes corresponding to said adjacent electrodes form an apex at an intersection region.

9. The GUI according to any one of claims 6 to 8, wherein said contact lobes are configured to expand asymmetrically with respect to a central axis of the catheter, so as to predominantly extend in a transverse direction towards the tissue wall.

10. The GUI according to any one of claims 6 to 9, wherein said contact lobes comprise ellipsoidal segments (440); and, the GUI is configured to compute, for each electrode, a variable transverse radius of said ellipsoidal segments, said variable transverse radius being based on the contact level between the tissue wall and said electrode and extending in a direction transverse to a local orientation of the catheter around said electrode.

11. A computer system (600) comprising at least one processing circuitry, configured to execute a method of providing real-time visual feedback of a contact level between a tissue wall of a luminal organ and an ablation catheter comprising a plurality of electrodes forming an elongated electrode array placed along a catheter distal assembly (510), the method comprising:

while the catheter distal assembly is in the luminal organ of a patient:

(a) rendering on a display a graphical representation of the catheter distal assembly and the plurality of electrodes thereon;
(b) for each electrode of the plurality of electrodes:

> i. repeatedly assessing tissue proximity of each of the plurality of electrodes; and
> ii. dynamically updating visual features indicative of the tissue proximity, said visual features comprising contact lobes (520, 530, 540), wherein
>
> > each contact lobe is centered on a corresponding electrode (515) and overlaid on said graphical representation of the catheter distal assembly;
> > a size of said contact lobe increases with the contact level between said tissue wall and said corresponding electrode, based on the tissue proximity.

12. The computer system according to claim 11, wherein assessing tissue proximity comprises measuring impedance of at least one electrode.

13. The computer system according to any one of claims 11 to 12, wherein when the tissue proximity of adjacent electrodes is above a predefined threshold, the contact lobes corresponding to said adjacent electrodes form an apex at an intersection region.

14. The computer system according to any one of claims 11 to 13, wherein said contact lobes are configured to expand asymmetrically with respect to a central axis of the catheter, so as to predominantly extend in a transverse direction towards the tissue wall.

15. The computer system according to any one of claims 11 to 14, wherein said contact lobes comprise ellipsoidal segments (440); and wherein the method comprises, for each electrode, computing a variable transverse radius of said ellipsoidal segments, said variable transverse radius being based on the contact level between the tissue wall and said electrode and extending in a direction transverse to a local orientation of the catheter around said electrode.

**Fig. 1A**

Fig. 1B

Fig. 1C

**Fig. 2A**

**Fig. 2B**

**Fig. 2C**

**Fig. 2D**

270

**Fig. 2E**

283
282

**Fig. 2F**

332
320
342
310
331
341a 343a
333
341b
341
343b 343
343

**Fig. 3A**

343a
341a
365
367a
367b
341b
343b

**Fig. 3B**

**Fig. 3C**

**Fig. 3D**

**Fig. 3E**

3740

3730

3720

**Fig. 4A**

440

$r_1$

$r_2$

420

450

430

$x$

460

450

470

445

475

457    455

**Fig. 4B**

## Fig. 5A

## Fig. 5B

## Fig. 5C

## Fig. 5D

| Organ rendering unit 607 | Catheter rendering unit 605 |
|---|---|
| Impedance measuring unit 612 | Tissue proximity unit 610 |
| Position measuring unit 615 | Size unit 625 |
| Intersection unit 630 | Apex unit 635 |
| Local radii unit 640 | Vertex unit 650 |
| Infill unit 670 | Color unit 660 |
| Display 690 | GPU 680 |

Visualization system

600

**Fig. 6**

Europäisches
Patentamt

European
Patent Office

Office européen
des brevets

# EUROPEAN SEARCH REPORT

**Application Number**

EP 25 18 8715

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/359968 A1 (HAGFORS MARK [US] ET AL) 19 November 2020 (2020-11-19) * paragraphs [0024] - [0036] * * figures 1-3 * ----- | 1-15 | INV. A61B5/00 A61B5/0538 A61B5/06 A61B18/14 |
| X | US 2024/138906 A1 (MATALON NAOR [IL] ET AL) 2 May 2024 (2024-05-02) * paragraphs [0020] - [0031] * * figures 2, 3A-3D * ----- | 1-15 | |
| X | US 2024/108240 A1 (BULLEMER BETH C [US] ET AL) 4 April 2024 (2024-04-04) * paragraphs [0055] - [0061] * * figures 8, 12A-12C * ----- | 1-15 | |
| A | US 2024/203035 A1 (ZAIDES LEONID [IL] ET AL) 20 June 2024 (2024-06-20) * paragraph [0105] * ----- | 1-15 | |

**TECHNICAL FIELDS SEARCHED (IPC)**

A61B

The present search report has been drawn up for all claims

| Place of search | Date of completion of the search | Examiner |
|---|---|---|
| The Hague | 25 August 2025 | Faymann, Juan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 18 8715

This annex lists the patent family members relating to the patent documents cited in the above-mentioned European search report.
The members are as contained in the European Patent Office EDP file on
The European Patent Office is in no way liable for these particulars which are merely given for the purpose of information.

25-08-2025

| Patent document cited in search report | Publication date | Patent family member(s) | | Publication date |
|---|---|---|---|---|
| US 2020359968 A1 | 19-11-2020 | EP | 3675729 A1 | 08-07-2020 |
| | | US | 2020359968 A1 | 19-11-2020 |
| | | WO | 2019046250 A1 | 07-03-2019 |
| US 2024138906 A1 | 02-05-2024 | CN | 120112235 A | 06-06-2025 |
| | | EP | 4608310 A1 | 03-09-2025 |
| | | IL | 320438 A | 01-06-2025 |
| | | US | 2024138906 A1 | 02-05-2024 |
| | | WO | 2024089527 A1 | 02-05-2024 |
| US 2024108240 A1 | 04-04-2024 | CN | 119894446 A | 25-04-2025 |
| | | EP | 4598440 A1 | 13-08-2025 |
| | | US | 2024108240 A1 | 04-04-2024 |
| | | WO | 2024076488 A1 | 11-04-2024 |
| US 2024203035 A1 | 20-06-2024 | CN | 119301644 A | 10-01-2025 |
| | | IL | 317297 A | 01-01-2025 |
| | | US | 2024203035 A1 | 20-06-2024 |
| | | WO | 2024127164 A1 | 20-06-2024 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 55391199 B
- US 5443489 A
- US 5558091 A
- US 6172499 B
- US 6239724 B
- US 6332089 B
- US 6484118 B
- US 6618612 B
- US 6690963 B
- US 6788967 B
- US 6892091 B
- US 7536218 B
- US 7756576 B
- US 7848787 B
- US 7869865 B
- US 8456182 B